# EUROPEAN PATENT SPECIFICATION

(11) **EP 0 972 520 B1**
(45) Date of publication and mention of the grant of the patent: **17.08.2005**
(21) Application number: 98901044.2
(22) Date of filing: 29.01.1998
(51) Int. Cl.: A61K 38/18, A61K 47/26

(54) **FREEZE-DRIED COMPOSITION OF BONE MORPHOGENETIC PROTEIN HUMAN MP52**
GEFRIERGETROCKNETE ZUSAMMENSETZUNG DES MENSCHLICHEN,MORPHOGENEN KNOCHENPROTEINS MP52
COMPOSITION LYOPHILISEE DE MP52 HUMAINE DE PROTEINES MORPHOGENETIQUES OSSEUSES

(30) Priority: 30.01.1997 JP 1634997
(43) Date of publication of application: 19.01.2000
(73) Proprietor: Biopharm Gesellschaft zur biotechnologischen Entwicklung von Pharmaka mbH, 69115 Heidelberg (DE)
(72) Inventor: INAGAKI, Mitsuko, Iruma-shi, Saitama 358 (JP); ICHIKAWA, Hideki, Hoechst Marion Roussel Ltd., Kawagoe-shi, Saitama 350-11 (JP)
(74) Representative: Böhm, Brigitte, Dipl.-Chem. Dr.
(86) International application number: PCT/JP1998/000371
(87) International publication number: WO 1998/033514

(56) References cited:
- EP-A- 0 567 391
- EP-A- 0 597 101
- EP-A- 0 955 313
- WO-A-93/16099
- WO-A-96/29095
- WO-A-96/33215
- JP-T- 7 503 847
- US-A- 4 675 184
- US-A- 5 037 644
- US-A- 5 464 614
- US-A- 5 597 897

## Description

### FIELD OF THE INVENTION

The present invention relates to a lyophilized composition of bone morphogenetic factor human MP52 and a preparation process therefor. More specifically, this invention pertains to a lyophilized composition which contains bone morphogenetic factor human MP52 and mannitol and a preparation process therefor.

### BACKGROUND OF THE INVENTION

The cDNA of bone morphogenetic factor human MP52 was isolated for the first time in 1994 as an osteogenesis-related factor classified as a TGF-β superfamily (Biochem. Biophy. Res. Comm., Vol. 204, No. 2, 1994). Then, an advanced genetic engineering technology has made it possible to prepare bone morphogenetic factor human MP52 without impairing its bone morphogenetic activity (WO96/33215). Bone morphogenetic factor human MP52 is stored under a lyophilized condition. It is, however, accompanied with a drawback that a volume reduction (shrink) occurs during storage and cohesion of powders occurs at the time of reconstitution.

With a view to overcoming the above-described problems, amino acids, saccharides or polyhydric alcohols are used for BMP-2 which is a protein classified as the same TGF-β super-family and has properties closest to bone morphogenetic factor

### DISCLOSURE OF THE INVENTION

The present inventors have proceeded with an extensive investigation with a view to overcoming the above-described problems. As a result, it has been found that when mannitol is added to bone morphogenetic factor human MP52, followed by lyophilization, neither coloring or shrink is observed during the storage of the lyophilized product and cohesion does not occur at the time of reconstitution, leading to the completion of the present invention.

The present invention, therefore, provides a lyophilized human MP52 (JP-A No. HEI 6-508777 = US 5,597,897). EP 0 567 391 describes a biodegradable TGF-β delivery system for bone regeneration. Such system contains TGF-β stabilized in a composition by an organic acid buffer, to which additional components have been added. EP-A-0 597 101 describes a stabilized human monoclonal antibody preparation. US 5,464,614 describes a stabilized superoxide dismutase (SOD) composition, containing as additives cyclodextrin and either a sugar or a sugar alcohol.

The present inventors, therefore, attempted the application of additives known from related prior art to bone morphogenetic factor human MP52 but could not overcome the above problems. Described specifically, cohesion at the time of reconstitution was observed even if a neutral or basic amino acid such as alanine, valine or lysine was added to bone morphogenetic factor human MP52 in an amount of 0.5 to 2.5% prior to lyophilization. When a saccharide such as sucrose or dextran was added in an amount of 0.5 to 1%, followed by lyophilization, color development to pale yellowish green and shrink were observed from the lyophilized product. When a polyhydric alcohol such as sorbitol was added in an amount of 0.5 to 1%, followed by lyophilization, bone morphogenetic factor human MP52 was dissolved in the period of lyophilization, which made it impossible to prepare a lyophilized product. composition of bone morphogenetic factor human MP52 containing bone morphogenetic factor human MP52 and mannitol. As bone morphogenetic factor human MP52 in the present invention, bone morphogenetic factor human MP52 (which may hereinafter be called "rhMP52") which has been prepared by genetic engineering technology disclosed in WO96/33215 is preferably used. As mannitol, that prescribed as D-mannitol in the Japanese Pharmacopoeia is preferably used. Bone morphogenetic factor human MP52 and mannitol are preferably mixed at a weight ratio of 1 : 5 - 50.

The composition according to the present invention can be prepared by lyophilizing an aqueous mixture solution of bone morphogenetic factor human MP52 and mannitol by a conventional method. Described specifically, the composition of the present invention is available by adding a predetermined amount of mannitol to an aqueous solution of purified bone morphogenetic factor human MP52, mixing them, filtering the resulting aqueous mixture solution, filling the filtrate in a sterile vial and carrying out lyophilization.

The composition of the present invention is administered to a patient in an amount effective for therapeutic treatment after being dissolved in distilled water for injection or weak acid (about pH 3), for example, a hydrochloric acid solution or citric acid buffer, upon use.

The preferred amount of mannitol incorporated in the composition of the present invention was determined by a stability test. The stability test was conducted in accordance with the method described in the instruction for the standard operation procedure prepared based on the Japanese Pharmacopoeia XIII, where properties such as appearance and clarity of solution, electrophoresis and water content at the beginning time of the test and 3 months later and ectopic bone formation after 6 months were observed or measured.

As a result, no change in the properties such as appearance and clarity of solution were observed at the beginning time of the test and 3 months later. From the results of the measurement on the electrophoresis and water content, the preparation containing mannitol at the above-described weight ratio was stable both at the beginning time of the test and 3 months later.

It is said that the preferred water content of a lyophilized product is generally 2% or lower. It was judged from the above findings that a bone morphogenetic factor human MP52 composition containing mannitol in an amount of 5 to 50 mg, desirably 10 mg, per 1 mg of bone morphogenetic factor human MP52 is preferred as a pharmaceutical product.

In addition, the ectopic bone formation of each of lyophilized compositions of bone morphogenetic factor human MP52 containing 10, 25 and 50 mg of mannitol, respectively was measured after stored for 6 months. As a result, ectopic bone formation was observed from any composition regardless of the storage temperature or amount of mannitol. Based on the above-described test results, it has been confirmed that the addition of mannitol to bone morphogenetic factor human MP52 prior to lyophilization does not have adverse effects on the bone morphogenetic factor human MP52 and the resulting composition remains stable for a long period of time.

### BEST MODE FOR CARRYING OUT THE INVENTION

The present invention will hereinafter be described more specifically by the following examples.

### Example 1 Preparation of an rhMP52 composition

To 1 mg/ml of an aqueous solution of purified rhMP52, which had been obtained by the preparation process disclosed in WO96/33215. D-mannitol of the Japanese Pharmacopoeia was added in an amount of 10, 25 and 50 mg, respectively, and they were mixed. After the resulting mixture was filtered through a 0.22 µm membrane filter, 1 ml portions of the filtrate so obtained were filled in vials sterilely. They were lyophilized, whereby a composition of the present invention was prepared in the form of pharmaceutical product.

### Example 2 Stability test of the rhMP52 composition

The rhMP52 lyophilized composition obtained in Example 1 was filled in a vial bottle (air-tight, transparent) and was stored at 2 - 8°C, 25° C and 40° C, respectively. The stability was evaluated based on the following criteria after three-month storage. The criteria for evaluation are as follows:

### Properties:

(Appearance): The composition which remained in the form of a white cake and was not colored was judged as "not changed".
(Clarity of solution): A solution having the composition dissolved in 1 ml of distilled water for injection was judged as "not changed" when it was colorless, transparent and cohesion-free.
(Electrophoresis): A purity of a main band was calculated from an area percentage, after introducing a picture by a film scanner which adopts transmission using a red film and then finding an integration optical density (IOD) % of each band.
(Water content): The water content of the composition was measured by a micro-moisture meter.

As a result, concerning properties, the composition of the present invention remained unchanged in appearance and clarity of solution, whereas cohesion was observed in a lyophilized product of rhMP52 alone. As a result of electrophoresis, each composition showed good stability (%).

The measurement results of water content (%) are shown in Table 1 which shows that there is not a large change in the water content (%) among the compositions. Table 1 shows that compared with the product composed only of rhMP52, the water content of each of the compositions according to the present invention is lower at the time of preparation or after storage and therefore shrink does not occur easily.

**Table 1**

| composition | Initial | 3 months later | | |
|---|---|---|---|---|
| | | 2 - 8°C | 25°C | 40°C |
| rbMP52 alone | 9.7% | 9.2% | 7.6% | 7.8% |
| | | | | |
| + Mannitol, 10 mg | 1.6% | 1.6% | 1.4% | 1.2% |
| | | | | |
| + Mannitol, 25 mg | 0.9% | 0.7% | 0.6% | 0.5% |
| | | | | |
| + Mannitol, 50 mg | 0.6% | 0.5% | 0.4% | 0.4% |

### Example 3 Ectopic bone formation of rhMP52

One vial (1 mg/vial) containing the rhMP52 composition obtained in Example 1 was stored at 4°C and 25°C for 6 months, respectively, then, 1 ml of distilled water for injection was added to the vial, whereby a solution for administration was prepared. The solution thus prepared was intramuscularly administered to an ICR mouse (purchased from Nippon Crea Co., Ltd.) in an amount of 20 µg/20 µl. Two weeks later, the presence or absence of ectopic bone formation was observed through a soft X-ray photographing (n=2). The results are shown in Table 2.

**Table 2**

| composition | 4°C | 25°C |
|---|---|---|
| rhMP52 alone | ebf* was observed | ebf was observed |
| | | |
| + Mannitol, 10 mg | ebf was observed | ebf was observed |
| | | |
| + Mannitol, 25 mg | ebf was observed | ebf was observed |
| | | |
| + Mannitol, 50 mg | ebf was observed | ebf was observed |
| ebf: ectopic bone formation | | |

### INDUSTRIAL APPLICABILITY

The lyophilized product of bone morphogenetic factor human MP52 involves problems that it is colored or it shrinks during storage and it coheres at the time of reconstitution. The lyophilized composition according to the present invention, however, is free from such problems. Bone morphogenetic factor human MP52 in the lyophilized composition of the present invention remains stable and no substantial change is observed in purity, water content and ectopic bone formation even after storage for a long time. Thus, the present invention is applicable in the field of pharmaceutical product.

## Claims

1. A reconstituted composition of bone morphogenetic factor human MP52 derived from a lyophilized composition, which comprises a mixture of bone morphogenetic factor human MP52 with mannitol at a mixing ratio in the range of 1:5-50 (ratio by weight).

2. The reconstituted composition according to claim 1, wherein said bone morphogenetic factor human MP52 has been produced by genetic engineering technology.

3. A process for the preparation of a reconstituted composition of bone morphogenetic factor human MP52 derived from a lyophilized composition, which comprises adding mannitol to an aqueous solution of purified bone morphogenetic factor human MP52 in an amount to give a mixing ratio of bone morphogenetic factor human MP52 to mannitol in the range of 1:5-50 (ratio by weight) and then lyophilizing the resultant mixed solution, and reconstituting the lyophilized composition.

4. The process according to claim 3, wherein said bone morphogenetic factor human MP52 has been produced by genetic engineering technology.

## Patentansprüche

1. Rekonstituierte Zusammensetzung des humanen knochenmorphogenen Faktors MP52, abgeleitet von einer lyophilisierten Zusammensetzung, welche ein Gemisch aus dem humanen knochenmorphogenen Faktor MP52 und Mannitol in einem Mischungsverhältnis im Bereich von 1 : 5 - 50 (Verhältnis nach Gewicht) umfasst.

2. Rekonstituierte Zusammensetzung nach Anspruch 1, wobei der humane knochenmorphogene Faktor MP52 durch gentechnologische Verfahren hergestellt wurde.

3. Verfahren zur Herstellung einer rekonstituierten Zusammensetzung des humanen knochenmorphogenen Faktors MP52, erhalten aus einer lyophilisierten Zusammensetzung, umfassend Hinzufügen von Mannitol zu einer wässrigen Lösung des aufgereinigten humanen knochenmorphogenen Faktors MP52 in einer Menge, um ein Mischungsverhältnis von humanem knochenmorphogenen Faktor MP52 zu Mannitol im Bereich von 1 : 5 - 50 (Verhältnis nach Gewicht) zu ergeben, und anschließendes Lyophilisieren der resultierenden vermischten Lösung, und Rekonstituieren der lyophilisierten Zusammensetzung.

4. Verfahren nach Anspruch 3, wobei der humane knochenmorphogene Faktor MP52 durch gentechnologische Verfahren hergestellt wurde.

## Revendications

1. Composition reconstituée de facteur morphogénétique osseux MP52 humain dérivée d'une composition lyophilisée, qui comprend un mélange de facteur morphogénétique osseux MP52 humain avec du mannitol, selon un rapport de mélange dans la gamme de 1 : 5-50 (rapport en poids).

2. Composition reconstituée selon la revendication 1, dans laquelle ledit facteur morphogénétique osseux MP52 humain a été produit par une technologie d'ingénierie génétique.

3. Procédé pour la préparation d'une composition reconstituée d'un facteur morphogénétique osseux MP52 humain dérivée d'une composition lyophilisée, qui comprend l'addition de mannitol à une solution aqueuse de facteur humain MP52 morphogénétique osseux purifié en une quantité pour donner un rapport de mélange du facteur morphogénétique osseux MP52 humain au mannitol dans la gamme de 1 : 5-50 (rapport en poids) et ensuite la lyophilisation de la solution mélangée résultante, et la reconstitution de la composition lyophilisée.

4. Procédé selon la revendication 3, dans lequel ledit facteur morphogénétique osseux MP52 humain a été produit par une technologie d'ingénierie génétique.
